# FASCICULE DE BREVET EUROPEEN

(11) **EP 2 643 472 B1**
(45) Date de publication et mention de la délivrance du brevet: **02.03.2016**
(21) Numéro de dépôt: 11799764.3
(22) Date de dépôt: 22.11.2011
(51) Int. Cl.: C12Q 1/04, G01N 33/569, C12M 1/34

(54) **DISPOSITIF DE DETECTION D'UNE CONTAMINATION FONGIQUE**
VORRICHTUNG FÜR DEN NACHWEIS EINER PILZKONTAMINATION
DEVICE FOR DETECTING A FUNGAL CONTAMINATION

(30) Priorité: 23.11.2010 FR 1059636
(43) Date de publication de la demande: 02.10.2013
(73) Titulaire: Centre Scientifique Et Technique Du Batiment (CSTB), 77420 Champs sur Marne (FR)
(72) Inventeur: MOULARAT, Stéphane, 77185 Lognes (FR); JOBLIN, Yaël, 94370 Sucy en Brie (FR); ROBINE, Enric, 77400 Lagny Sur Marne (FR)
(74) Mandataire: Cabinet Plasseraud
(86) Numéro de dépôt international: PCT/FR2011/052720
(87) Numéro de publication internationale: WO 2012/069752

(56) Documents cités:
- WO-A1-2008/125770
- Staples EJ: "The zNose: A New Electronic Nose Using Acoustic Technology", The Journal of the Acoustical Society of America, no. Paper No: 2aEA4 décembre 2000 (2000-12), pages 1-8, XP002638660, City of Publication: Publishing organization; 2000 Extrait de l'Internet: URL:http://www.aromako.co.kr/aroma/tech/pd f/asa2000.pdf [extrait le 2011-05-24]
- CASALINUOVO I A ET AL: "Experimental use of a new surface acoustic wave sensor for the rapid identification of bacteria and yeasts.", LETTERS IN APPLIED MICROBIOLOGY JAN 2006 LNKD- PUBMED:16411915, vol. 42, no. 1, janvier 2006 (2006-01), pages 24-29, XP002638661, ISSN: 0266-8254
- Anonymous: "( BW)(CA-ELECTRONIC-SENSOR) zNose, an `Eye in the Kingdom of the Blind' for the Chemical World", Electronic Sensor Technology , 7 novembre 2001 (2001-11-07), pages 1-2, XP002638662, Extrait de l'Internet: URL:http://www.estcal.com/press_releases/a rticles/zNose%20Eye%20for%20Blind_files/f_ headline.htm [extrait le 2011-05-24]
- Jonas Holme: "Detection, assessment and eva luation of mould in buildings in relation to indoor environment and effects on human health", Report from the R&D-programme «Climate 2000» , octobre 2006 (2006-10), pages FP,1-40, XP002638663, ISSN: 0801-6461 Extrait de l'Internet: URL:http://www.sintef-group.com/upload/Byg gforsk/Publikasjoner/Prrapp%20406.pdf [extrait le 2011-05-24]
- JOBLIN Y ET AL: "Detection of moulds by volatile organic compounds: Application to heritage conservation", INTERNATIONAL BIODETERIORATION AND BIODEGRADATION, vol. 64, no. 3, 1 juin 2010 (2010-06-01), pages 210-217, XP026990854, ELSEVIER LTD, GB ISSN: 0964-8305 [extrait le 2010-02-20]
- S. HAMILTON A ET AL: "Detection of Serpula lacrymans infestation with a polypyrrole sensor array", SENSORS AND ACTUATORS B, vol. 113, no. 2, 27 février 2006 (2006-02-27), pages 989-997, XP002638664, DOI: 10.1016/j.snb.2005.04.014

## Description

La présente invention concerne un dispositif de détection d'une contamination fongique dans un environnement intérieur, son utilisation ainsi qu'un procédé de détection d'une contamination fongique dans un environnement intérieur mettant en oeuvre un tel dispositif.

Par environnement intérieur on entend un espace confiné à l'intérieur d'un bâtiment qui est aéré de façon non continu. Des exemples d'environnements intérieurs peuvent être trouvés dans les habitations, les musées, les églises, les caves, les monuments historiques, les bâtiments administratifs, les écoles et les hôpitaux.

La présence de moisissures dans les environnements intérieurs n'est pas sans conséquences sanitaires. En effet, de nombreuses études ont démontré l'apparition de symptômes chez les occupants de locaux comportant des moisissures, et également leur rôle dans la dégradation à la fois des matériaux et des ouvrages qu'elles colonisent. En effet, les enzymes et/ou les acides produits par les champignons provoquent également la détérioration de leur support.

Les techniques de détection de présence de moisissures dans les environnements intérieurs reposant sur la reconnaissance visuelle d'un développement fongique et la mise en culture de conidies prélevées dans l'air ou sur les surfaces ne permettent pas de déceler efficacement les contaminations « cachées » ni de détecter les contaminations assez tôt pour prévenir efficacement la détérioration de leurs supports. En effet, cette détérioration est généralement déjà avancée lorsque la contamination est détectable par reconnaissance visuelle. De plus, le temps de réponse de ces techniques de mesure est long puisqu'il est nécessaire d'attendre la croissance en laboratoire des microorganismes prélevés avant de pouvoir réaliser l'analyse. Par conséquent, il existe, en particulier pour certains domaines sensibles comme la préservation des oeuvres artistiques ou historiques, une demande pour une solution permettant une détection précoce et une surveillance en continu des contaminations fongiques.

Les champignons émettent dès le début de leur développement des molécules volatiles (Composés Organiques Volatils, COV) issues soit de leur métabolisme, soit de la dégradation du matériau sur lequel ils se développent par les enzymes ou les acides qu'ils produisent. Les COV diffusent à travers les parois et peuvent être détectés dans l'air même dans le cas de contaminations cachées. Cependant, les COV présents dans un environnement intérieur peuvent également provenir d'autres sources telles que les matériaux de construction, les produits ménagers ou encore l'activité humaine. Les concentrations des COV d'origine fongique, notamment à un stade précoce de contamination, s'avèrent être relativement faibles comparées à l'ensemble des COV présents dans un environnement intérieur.

La demande de brevet FR 2913501 propose un procédé de détection d'une contamination fongique dans un environnement intérieur par la détermination d'un indice de contamination fongique basée sur l'analyse des COV présents dans l'air ambiant. Ce procédé permet la détection d'un développement fongique à un stade précoce de son développement même en cas de contamination cachée mais met en oeuvre des méthodes classiques d'analyse, comme la chromatographie en phase gazeuse couplée à un spectromètre de masse. Ces méthodes nécessitent la collecte d'un échantillon qui doit être rapporté au laboratoire où il subira de longues étapes de concentration, de séparation et d'analyse. Ces étapes pour la détection d'une contamination fongique dans un environnement intérieur nécessitent l'intervention d'un technicien qualifié et se révèlent relativement longues et coûteuses. Ces techniques d'analyse ne permettent donc pas une mesure rapide et en continu.

Les capteurs chimiques sont couramment utilisés pour la mesure en continu des polluants organiques. Cependant, de tels capteurs ne sont pas suffisamment sensibles pour détecter les niveaux de concentrations en COV émis lors d'un développement fongique, ni suffisamment sélectifs pour différencier ces COV d'origine fongique des autres COV provenant d'autres sources biologiques ou des matériaux de construction.

Le document Casalinuovo et al, Letters in Applied Microbiology 42 (2006) 24-29, décrit toutefois l'utilisation d'un capteur chimique, zNose^{™} pour la détection bactérienne et fongique.

Les solutions disponibles à ce jour ne permettent donc pas de répondre à la demande de détection précoce et de surveillance en continu des contaminations fongiques.

La société demanderesse a quant à elle réussi à mettre au point un dispositif de détection d'une contamination fongique dans un environnement intérieur permettant une analyse rapide *in situ* de l'air ambiant avec un faible temps de mesure, et donc la détection de contamination en continu. Le dispositif de l'invention présente de plus l'avantage de pouvoir être utilisé sans l'intervention d'un technicien spécialisé.

Ainsi, la présente invention concerne un dispositif de détection d'une contamination fongique dans un environnement intérieur comprenant :
- un module de préconcentration ;
- un module de séparation comprenant une micro-colonne chromatographique; et
- un module de détection comprenant une matrice de capteurs.
caractérisé en ce que le module de séparation comprend un système de sélection de COV cibles.

La présence ou l'absence de moisissure dans un environnement intérieur ne peut pas être déduite à partir de la détection d'un seul COV d'origine fongique. Les présents inventeurs ont donc conçu un dispositif qui utilise un principe de détection d'une contamination fongique reposant sur la détection de certains COV cibles. Le dispositif de l'invention permet donc en particulier la détection de la présence ou l'absence de COV cibles choisis parmi une palette de COV cibles pouvant résulter du développement d'une contamination fongique. Les COV cibles comprennent notamment :
(1) les COV qui sont émis indépendamment de l'espèce fongique et de leur support et qui ne sont émis que par des espèces fongiques, tels que le 1-octèn-3-ol, le 1,3-octadiène et le méthyl-2-éthylhexanoate ;
(2) les COV qui sont émis indépendamment de l'espèce fongique et du support, mais qui peuvent également avoir d'autres origines biologiques, tels que le 2-méthylfurane, le 3-méthylfurane, le 3-méthyl-1-butanol, le 2- méthyl-1-butanol et le α-pinène ;
(3) les COV qui sont émis en fonction de l'espèce fongique et/ou du support, tels que le 2-heptène, le diméthylsulfure, le 4-heptanone, le 2(5H)-furanone, le 3-heptanol et le méthoxybenzène.

Les COV cibles peuvent également comprendre des COV n'appartenant pas aux catégories (1), (2) ou (3) mais intervenant dans l'appréciation de la présence d'une contamination fongique, tel que le 2-éthylhexanol.

Le module de préconcentration du dispositif selon l'invention permet une concentration des COV cibles présents dans l'air ambiant jusqu'à une concentration détectable par le module de détection. La concentration des COV peut être réalisée par toute méthode connue de l'homme du métier, en particulier l'accumulation sur un matériau adsorbant. Le module de préconcentration comprend donc avantageusement un matériau adsorbant permettant l'accumulation des COV cibles. La structure du matériau adsorbant possède typiquement une forme permettant d'optimiser sa surface spécifique. De préférence, le matériau adsorbant est sous forme de particules ayant typiquement une taille de 50 à 200 µm, une surface spécifique de 20 à 50 m²/g, une porosité de 1 à 5 cm³/g et une taille de pores moyenne de 50 à 500 nm. Le matériau adsorbant est préférentiellement choisi parmi le charbon actif, le gel de silice, les zéolithes et les résines synthétiques poreuses, tels ceux commercialisés sous la marque Tenax®, Carbograph® ou Chromosorb®. Le module de préconcentration comprend avantageusement en outre un système de chauffage permettant la désorption des COV adsorbés sur le matériau adsorbant.

Selon un mode de réalisation particulier du dispositif de l'invention, le module de préconcentration comprend un micro-préconcentrateur. Un tel micro-préconcentrateur présente typiquement un volume utile de 0,1 à 1 cm³, de préférence 0,1 à 0,5 cm³, plus préférentiellement de 0,1 à 0,3 cm³. Le micro-préconcentrateur est constitué d'une plaque de substrat, telle qu'une plaque de silicium, sur la surface duquel sont gravés des sillons dans lesquels se trouve le matériau adsorbant. Une seconde plaque, d'un matériau identique ou différent du substrat (tel qu'une plaque de verre), collée sur la surface de la plaque de substrat gravée comportant les sillons, referme le micro-préconcentrateur. La plaque de substrat a typiquement une surface de 5 à 20 cm². Les sillons ont avantageusement une longueur de 3 à 10 cm, une largeur de 200 à 1000 µm, une profondeur de 200 à 1000 µm, et une section de 0,04 à 1 mm². La section des sillons peut présenter diverses formes telles que rectangulaire, semi-circulaire ou circulaire.

Avantageusement, le module de préconcentration comprend également un système de circulation forcée permettant de forcer le passage de l'air ambiant à travers le module de préconcentration.

Le module de séparation comprend une micro-colonne chromatographique ayant avantageusement une section de 0,01 à 0,25 mm². La longueur de la micro-colonne doit être également choisie de sorte à optimiser la séparation des COV. Elle est avantageusement supérieure à 1 m, de préférence comprise entre 1 et 50 m. Le choix d'une longueur importante permet d'améliorer l'efficacité de la colonne et donc d'obtenir une meilleure séparation des COV. La micro-colonne comprend une phase stationnaire que l'homme du métier saura choisir de sorte à optimiser la séparation des COV. Celle-ci appartient avantageusement à la famille des polysiloxanes (par exemple le diméthylpolysiloxane (PDMS)). Différentes phases stationnaires peuvent également être utilisées. Ces phases peuvent être des hydrocarbures ramifiés, des polyéthylène glycols et polypropylène glycols, des polyesters, des sulfones de polyaryléthers, ou encore des phases stationnaires à sélectivités spécifiques.

La micro-colonne comprend par exemple une plaque de substrat, tel qu'une plaque de silicium, sur la surface duquel est gravé un sillon dans lequel se trouve la phase stationnaire. Une seconde plaque, d'un matériau identique ou différent du substrat (telle qu'une plaque de verre), collée sur la surface de la plaque de substrat gravée comportant le sillon, referme la micro-colonne. La plaque de substrat a typiquement une surface de 5 à 20 cm². Le sillon a avantageusement une longueur de plus de 1 m, de préférence de 1 à 50 m, une largeur de 100 à 500 µm, une profondeur de 100 à 500 µm, et une section de 0,01 à 0,25 mm². La section des sillons peut présenter diverses formes telles que rectangulaire, semi-circulaire ou circulaire. Le sillon peut être agencé de différentes manières de sorte à minimiser l'encombrement et donc la taille de la structure, par exemple en lacets parallèles (serpentin).

Le module de séparation comprend également un système de sélection des COV cibles comprenant de préférence une électrovanne et une unité programmable permettant le contrôle de ladite électrovanne. Ce système de sélection est directement connecté à la sortie de la micro-colonne. Le temps de rétention, pour une phase stationnaire et une longueur de micro-colonne données, est spécifique pour chaque COV. Ainsi, en renseignant les temps de rétention de chaque COV cible, l'unité programmable peut être préprogrammée de sorte que le système de sélection dirige sélectivement les portions d'éluât correspondantes aux temps de rétention de chaque COV cible vers le module de détection, le reste de l'éluât étant évacué du circuit d'analyse. Lesdites portions d'éluât peuvent être soit envoyées les unes après les autres au module de détection, au fur et à mesure de l'élution, soit stockées puis envoyées ensemble dans le module de détection.

Les COV cibles, comprenant principalement des COV d'origine fongique, ont des concentrations très faibles comparées aux concentrations totales de l'ensemble des COV présents dans l'air ambiant. Ainsi, cette séparation sélective des COV cibles permet d'empêcher la formation d'un bruit de fond et/ou les phénomènes d'hystérésis et/ou de saturation des capteurs du module de détection qui seraient préjudiciables à la détection des COV cibles.

Le module de détection du dispositif selon l'invention comprend une matrice de capteurs avantageusement choisis parmi les capteurs électrochimiques de type polymère ou de type oxyde métallique. Les capteurs comprennent de préférence une couche de polymère ou de mélange de polymères ayant une affinité avec les COV d'origine fongique. Le polymère peut être choisi parmi les polypyrroles, les polythiophènes, les polyanilines, et leurs dérivés. En particulier, la sensibilité du polybifluorène, du poly(3,4-éthylènedioxythiophène)/poly(styrène sulfonate) de sodium (PEDOT-PSS), du polypyrrole/octane sulfonate de sodium et du polypyrrole/perchlorate de lithium à un environnement fongique a été démontrée.

Les COV peuvent être classés en différentes familles selon leur nature chimique : les COV aliphatiques, les alcools, les cétones, les esters, les éthers, les aldéhydes, les COV aromatiques, les COV chlorés, les COV azotés ou les COV soufrés. Il existe des capteurs chimiques permettant la détection de composés possédant un groupe fonctionnel déterminé. De tels capteurs permettent de détecter et d'identifier la présence d'un COV appartenant à une famille déterminée mais ne permettent pas de différencier des COV appartenant à une même famille.

Dans un mode de réalisation particulier, la matrice de capteurs comprend des capteurs spécifiques à chaque famille de COV. Dans ce cas, la réponse de la matrice de capteurs permet de conclure sur la présence ou l'absence d'un COV dans une portion d'éluât donnée, mais ne suffit pas à elle seule à déterminer la nature du COV détecté. En revanche, la réponse de la matrice de capteurs permet de déterminer à quelle(s) famille(s) appartient le COV détecté et la connaissance du temps de rétention de la portion d'éluât considérée permet de savoir quel COV cible peut être présent dans ladite portion d'éluât. Ainsi, il est possible de déduire la présence ou l'absence de chaque COV cibles en combinant les informations fournies par le temps de rétention et la matrice de capteurs.

Dans un autre mode de réalisation, la matrice comprend un ensemble de capteurs permettant l'obtention d'une empreinte globale spécifique à chaque COV cible. Par empreinte globale, on entend la combinaison des réponses de l'ensemble des capteurs de la matrice. Dans ce cas, bien que chaque capteur de la matrice ne soit pas spécifique à un seul COV cible, la réponse combinée de plusieurs capteurs permet d'identifier de manière spécifique chaque COV cible. Ainsi, il est possible de déduire la présence ou l'absence de chaque COV cible à partir des informations fournies par la matrice de capteurs.

Dans un autre mode de réalisation, la matrice de capteurs comprend des capteurs spécifiques à chaque COV cible. Dans ce cas, la matrice de capteurs comprend autant de capteurs que de COV cibles et la réponse de chaque capteur spécifique permet de conclure individuellement sur la présence ou l'absence du COV cible dont il est spécifique.

Avantageusement, le module de détection comprend également une chambre de confinement enfermant la matrice de capteurs. Cette chambre permet le confinement des couches sensibles des capteurs afin de les exposer uniquement aux échantillons à analyser. Avantageusement, la chambre de confinement est réalisée dans un matériau non émissif ou peu émissif de COV dans les conditions d'analyse, tel que le l'acier inoxydable ou le polytétrafluoroéthylène (PTFE), afin d'éviter la contamination de l'échantillon à analyser.

Dans un mode de réalisation particulier, le dispositif de l'invention comprend en outre un module de traitement de l'information. Celui-ci est capable d'interpréter les signaux émis par chaque capteur et de déduire la présence ou l'absence de chaque COV cible. De préférence, le module de traitement de l'information détermine la présence ou non d'une contamination fongique. Cette détermination peut se faire par exemple par le calcul d'un indice de contamination fongique tel que défini dans la demande de brevet FR 2913501.

Les méthodes classiques de détection et/ou d'identification mettent en oeuvre des appareillages complexes tels que des spectromètres de masse, des spectromètres infrarouge, des détecteurs à ionisation de flamme ou des détecteurs à conductivité thermique qui sont difficiles à miniaturiser. L'originalité du dispositif de l'invention réside dans le couplage d'une micro-colonne chromatographique avec des capteurs chimiques. Ce dispositif a l'avantage de pouvoir être miniaturisé et de pouvoir être utilisé sans l'intervention d'un technicien spécialisé.

Le dispositif de l'invention présente donc un avantage quant à sa taille et à son autonomie, ce qui permet de réduire considérablement l'intervalle de temps entre les mesures successives et/ou le temps de réponse de la mesure. La durée d'une mesure avec le dispositif de l'invention est typiquement de 10 à 180 min, de préférence 30 à 120 min. Un tel dispositif offre donc la possibilité de mettre en place une stratégie efficace de surveillance des contaminations fongiques avec un intervalle de temps faible entre les mesures. Ainsi une procédure d'alerte peut être envisagée afin de rechercher et traiter les contaminations à leurs premiers stades de développement. De plus, les systèmes de contrôle de l'air ambiant, tels que les VMC, peuvent être asservis au dispositif de l'invention pour empêcher ou limiter le développement fongique.

La présente invention concerne également un procédé de détection d'une contamination fongique dans un environnement intérieur mis en oeuvre par le dispositif de l'invention et comprenant:
- le prélèvement d'un échantillon de COV dans l'environnement intérieur ;
- la séparation des COV prélevés ;
- la sélection de COV cible ; et
- la détection des COV présents.

Le procédé de l'invention comprend le prélèvement d'un échantillon de COV dans l'environnement intérieur. Pour ce faire, le dispositif de l'invention est disposé dans l'environnement intérieur et le prélèvement est effectué par contact entre le module de préconcentration et l'air ambiant. Dans une première alternative, le prélèvement est effectué par convection naturelle de l'air ambiant. Le prélèvement de l'échantillon dure alors entre 60 et 300 min. Dans une alternative préférée, le prélèvement est effectué par convection forcée provoquant le passage de l'air ambiant au travers du module de préconcentration. Le débit de l'air ambiant passant au travers du module de prélèvement est par exemple de 10 à 1000 mL/min. Le prélèvement de l'échantillon dure alors entre 5 et 60 min. Le prélèvement est de préférence effectué par adsorption des COV sur un matériau adsorbant. Dans ce cas, le procédé de l'invention comprend également une étape de désorption des COV adsorbés. Celle-ci est effectuée par thermo-désorption dans des conditions bien connues de l'homme du métier.

Le procédé de l'invention comprend également la séparation des COV prélevés. La séparation des COV prélevés est effectuée par le module de séparation. En particulier, les COV prélevés sont séparés par élution sur une micro-colonne chromatographique. Les paramètres optimums de séparation comme la température de la colonne ou le débit de la phase mobile, sont déterminés selon les techniques bien connues de l'homme du métier en fonction de la géométrie de la colonne, de la nature de la phase stationnaire et du gaz vecteur.

Le procédé de l'invention comprend également la détection des COV présents. Au fur et à mesure de l'élution, l'éluât est dirigé vers le module de détection où la détection des COV présents est effectuée grâce à l'analyse de l'éluât par la matrice de capteurs.

Le procédé de l'invention comprend également la sélection des COV cibles parmi les COV prélevés par le module de séparation. Cette étape est effectuée par le système de sélection au cours de l'élution de l'échantillon sur la micro-colonne chromatographique. Pour ce faire, on procède de la façon suivante. Chaque COV cible élue à une vitesse différente connue pour un système chromatographique donné. On attribue donc un temps de rétention donné à un COV cible. On programme le système de sélection avec ces valeurs. Le système de sélection est alors capable de sélectionner les portions d'éluât ayant un temps de rétention correspondant aux COV cibles. Ces portions d'éluât sont alors envoyées sélectivement vers le module de détection. Les portions d'éluât ne correspondant pas aux valeurs préprogrammées sont éliminées. Par conséquent, seules sont détectées par le module de détection la présence ou l'absence des COV cibles.

Le reste de l'éluât étant évacué du circuit d'analyse, cela permet d'éviter les phénomènes d'hystérésis et/ou de saturation des capteurs du module de détection que pourrait provoquer la présence des COV non cibles qui ont généralement une concentration bien supérieure à celle des COV d'origine fongique.

Les COV cibles sont de préférence choisis dans le groupe consistant en le 1-octèn-3-ol, le 1,3-octadiène, le méthyl-2-éthylhexanoate, le 2-méthylfurane, le 3-méthylfurane, le 3-méthyl-1-butanol, le 2- méthyl-1-butanol, le α-pinène le 2-heptène, le diméthylsulfure, le 4-heptanone, le 2(5H)-furanone, le 3-heptanol, le méthoxybenzène et le 2-éthylhexanol, et leurs mélanges.

Avantageusement, le procédé de l'invention comprend également la détermination d'un indice de contamination fongique, par exemple en utilisant le procédé comme défini dans la demande de brevet FR 2913501.

Le procédé selon l'invention est de préférence utilisé en continu. Avantageusement, la durée d'un cycle de mesure est de 10 à 180 min, de préférence 30 à 120 min.

La présente invention concerne également l'utilisation du dispositif selon l'invention pour détecter une contamination fongique dans un environnement intérieur.

Le dispositif de l'invention peut être également utilisé dans un système de contrôle de l'air ambiant, tel qu'une VMC.

Les exemples de réalisation suivants illustrent la présente invention, sans en limiter en aucune façon la portée.

### EXEMPLE 1 : Réalisation du dispositif

Le module de préconcentration comprend un micro-préconcentrateur gravé sur une plaque de silicium par un procédé DRIE. Le micro-préconcentrateur est composée de 20 sillons de 6 cm de long, de section rectangulaire de 500 µm de largeur et 400 µm de longueur, et présente un volume utile de 0,25 m². Les sillons sont remplis de particules de résine à base d'oxyde de 2,6-diphényle commercialisé sous le nom TENAX® TA ayant un diamètre moyen de 120 µm, une surface spécifique de 35 m²/g, une porosité de 2,4 cm³/g et une taille de pores moyenne de 200 nm. Le micro-préconcentrateur est refermé par une plaque de silicium collée sur la surface comportant les sillons de la première plaque.

Une micro-colonne chromatographique a été gravée sur une plaque de silicium par un procédé DRIE. La micro-colonne est composée d'un sillon de 5 m de long, de section rectangulaire de 150 µm de largeur et 200 µm de longueur. Le sillon est agencé sous forme de lacets parallèles (ou serpentin) présentant des coudes sous forme d'arc de cercle afin d'éviter la formation d'angles morts. Une phase stationnaire de PDMS, diméthylpolysiloxane (Sylgard® 184, Dow corning), est présente à l'intérieur de la micro-colonne. La micro-colonne est refermée avec une seconde plaque de silicium collée sur la surface comportant le sillon de la première plaque.

Le module de détection comprend une matrice de capteurs composée de 4 capteurs polymères. Les capteurs polymères ayant une affinité avec les COV d'origine fongique (respectivement le PEDOT-PSS, le polypyrrole/octane sulfonate de sodium, le polypyrrole/perchlorate de lithium et le polybifluorène) déposé sur des paires d'électrodes interdigitées. La matrice de capteurs est disposée dans une chambre de confinement en PTFE.

Les différents éléments sont reliés entre eux et au système de circulation par des connecteurs NanoPort™.

### EXEMPLE 2 : Etalonnage de la micro-colonne

Pour l'étalonnage, la matrice de capteurs du dispositif de l'exemple 1 a été remplacée par un spectromètre de masse.

Les paramètres expérimentaux de la chaîne d'analyse sont rassemblés dans le tableau 1.

**Tableau 1 : Caractéristiques du GC/MS**

| Paramètres | Conditions analytiques |
|---|---|
| Thermo-désorbeur | Turbomatrix ATD (Perkin Elmer) |
| Température de désorption | 370 °C |
| Débit de désorption | 50 mL/min Azote N50 |
| Durée de désorption | 15 min |
| Température du piège froid (Tenax TA) | -30 °C |
| Température d'injection (40°C/s) | 300 °C |
| Température de la ligne de transfert | 220 °C |
| Chromatographe gaz / Spectromètre de masse | Autosystem XL / Turbomass (Perkin Elmer) |
| Microcolonne | Sylgard 184 |
| Gaz vecteur | Hélium N60 |
| Pression constante | 37,5 psi |
| Cycle de température | 40 °C pendant 2 min 1°C/min. jusqu'à 41°C Plateau de 2 min 0,3°C/min. jusqu'à 44°C pendant 2 min 1°C/min. jusqu'à 47°C Plateau de 2 min |
| Paramètres du spectromètre de masse | Quadripôle mode EI, balayage (33-400) |

Des échantillons des COV cibles ont été passés dans la micro-colonne afin de déterminer les temps de rétention de chaque COV cible.

Les temps de rétention de chaque COV cible sont répertoriés dans le tableau 2.

**Tableau 2**

| **Composés** | **Temps de rétention (min)** |
|---|---|
| 1-octen-3-ol | 4.7 |
| 1,3-octadiène | 1.6 |
| méthyl-2-éthylhexanoate | 9.1 |
| 2-éthylhexanol | 5.7 |
| α-pinène | 2.7 |
| 2-méthylfurane | 0.5 |
| 3-méthylfurane | 0.5 |
| 3-méthyl-1-butanol | 1.4 |
| 2-méthyl-1-butanol | 1.4 |
| 2-héptène | 0.8 |
| 4-heptanone | 2.1 |
| 3-heptanol | 4.8 |
| Méthoxybenzène | 2.6 |

### EXEMPLE 3 : Détection d'une contamination fongique

Le dispositif de l'exemple 1, comprenant une matrice de capteurs, a été placé dans différents environnements intérieurs sains ou présentant une contamination fongique à différents stades de développement.

Le prélèvement de COV dans l'environnement intérieur a été réalisé par convection forcée de l'air ambiant au travers du module de préconcentration à un débit de 100 mL/min pendant 15 minutes.

Les paramètres expérimentaux concernant la micro-colonne sont identiques à ceux de l'exemple 2.

La durée totale des mesures est de 20 min.

Les réponses de la matrice de capteurs a permis de détecter la présence ou l'absence de COV cible et un indice de contamination fongique tel que défini dans la demande de brevet FR 2913501 a également pu être calculé.

## Revendications

1. Dispositif de détection d'une contamination fongique dans un environnement intérieur comprenant :
- un module de préconcentration ;
- un module de séparation comprenant une micro-colonne chromatographique; et
- un module de détection comprenant une matrice de capteurs ;
**caractérisé en ce que** le module de séparation comprend un système de sélection de COV (Composés Organiques Volatiles) cibles.

2. Dispositif selon la revendication 1, **caractérisé en ce que** le module de préconcentration comprend un matériau adsorbant.

3. Dispositif selon la revendication 1 ou 2, **caractérisé en ce que** la micro-colonne a une longueur comprise entre 1 et 50 m.

4. Dispositif selon l'une quelconque des revendications 1 à 3, **caractérisé en ce que** la matrice de capteur comprend au moins un polymère ayant une affinité avec les COV d'origine fongique.

5. Procédé de détection d'une contamination fongique dans un environnement intérieur mis en oeuvre par le dispositif tel que défini dans les revendications 1 à 4 comprenant:
- le prélèvement d'un échantillon de COV dans l'environnement intérieur ;
- la séparation des COV prélevés ;
- la sélection de COV cibles ; et
- la détection des COV cibles présents.

6. Procédé selon la revendication 5, **caractérisé en ce que** les COV cibles sont choisis parmi le groupe consistant en le 1-octèn-3-ol, le 1,3-octadiène, le méthyl-2-éthylhexanoate, le 2-méthylfurane, le 3-méthylfurane, le 3-méthyl-1-butanol, le 2- méthyl-1-butanol, le α-pinène le 2-heptène, le diméthylsulfide, le 4-heptanone, le 2(5H)-furanone, le 3-heptanol, le méthoxybenzène et le 2-éthylhexanol, et leurs mélanges.

7. Procédé selon l'une quelconque des revendications 5 à 6, **caractérisé en ce que** la détermination de la contamination fongique se fait en continu.

8. Utilisation du dispositif tel que défini dans les revendications 1 à 4 pour détecter une contamination fongique dans un environnement intérieur.

## Patentansprüche

1. Vorrichtung für den Nachweis einer Pilzkontamination in einer geschlossenen Umgebung, umfassend:
- ein Vorkonzentrationsmodul;
- ein Trennmodul, umfassend eine chromatographische Mikrosäule; und
- ein Detektionsmodul, umfassend eine Sensormatrix;
**dadurch gekennzeichnet, dass** das Trennmodul ein System zur Selektion von Ziel-COV (flüchtige organische Verbindungen) umfasst.

2. Vorrichtung gemäß Anspruch 1, **dadurch gekennzeichnet, dass** das Vorkonzentrationsmodul ein Adsorptionsmaterial umfasst.

3. Vorrichtung gemäß Anspruch 1 oder 2, **dadurch gekennzeichnet, dass** die Mikrosäule eine Länge zwischen 1 und 50 m aufweist.

4. Vorrichtung gemäß einem beliebigen der Ansprüche 1 bis 3, **dadurch gekennzeichnet, dass** die Sensormatrix zumindest ein Polymer umfasst, das eine Affinität zu den COV pilzlichen Ursprungs aufweist.

5. Verfahren zur Detektion einer Pilzkontamination in einer geschlossenen Umgebung, das mit einer Vorrichtung wie in den Ansprüchen 1 bis 4 definiert ausgeführt wird, umfassend:
- die Entnahme einer Probe von COV in der geschlossenen Umgebung;
- die Trennung der entnommenen COV;
- die Selektion der Ziel-COV; und
- die Detektion der vorhandenen Ziel-COV.

6. Verfahren gemäß Anspruch 5, **dadurch gekennzeichnet, dass** die Ziel-COV aus der Gruppe ausgewählt sind, bestehend aus 1-Octen-3-ol, 1,3-Octadien, Methyl-2-ethylhexanoat, 2-Methylfuran, 3-Methylfuran, 3-Methyl-1-butanol, 2-Methyl-1-butanol, α-Pinen 2-Hepten, Dimethylsulfid, 4-Heptanon, 2(5H)-Furanon, 3-Heptanol, Methoxybenzen und 2-Ethylhexanol und ihren Mischungen.

7. Verfahren gemäß einem beliebigen der Ansprüche 5 bis 6, **dadurch gekennzeichnet, dass** die Bestimmung der Pilzkontamination kontinuierlich erfolgt.

8. Verwendung der Vorrichtung wie in den Ansprüchen 1 bis 4 definiert zum Detektieren einer Pilzkontamination in einer geschlossenen Umgebung.

## Claims

1. A device for detecting a fungal contamination in an internal environment comprising:
- a preconcentration module;
- a separation module comprising a chromatographic microcolumn; and
- a detection module comprising a matrix of sensors ;
**characterized in that** the separation module comprises a system for selecting target VOCs (volatile organic compounds).

2. The device as claimed in claim 1, **characterized in that** the preconcentration module comprises an adsorbent material.

3. The device as claimed in claim 1 or 2, **characterized in that** the microcolumn has a length between 1 and 50 m.

4. The device as claimed in any one of claims 1 to 3, **characterized in that** the matrix of sensors comprises at least one polymer having an affinity with the VOCs of fungal origin.

5. A method for detecting a fungal contamination in an internal environment which is implemented by the device as defined in claims 1 to 4 comprising:
- taking a sample of VOCs from the internal environment;
- separating the VOCs sampled;
- selecting target VOCs; and
- detecting the target VOCs present.

6. The method as claimed in claim 5, **characterized in that** the target VOCs are chosen from the group consisting of 1-octen-3-ol, 1,3-octadiene, methyl 2-ethylhexanoate, 2-methylfuran, 3-methylfuran, 3-methyl-1-butanol, 2-methyl-1-butanol, α-pinene, 2-heptene, dimethyl sulfide, 4-heptanone, 2(5H)-furanone, 3-heptanol, methoxybenzene and 2-ethylhexanol, and mixtures thereof.

7. The method as claimed in any one of claims 5 to 6, **characterized in that** the determining of the fungal contamination is carried out continuously.

8. The use of the device as defined in claims 1 to 4, for detecting a fungal contamination in an internal environment.
